(19) Europäisches Patentamt
European Patent Office
Office européen des brevets

(11) Publication number: **0 253 582**
**A1**

# (12) EUROPEAN PATENT APPLICATION

(21) Application number: **87306099.0**

(22) Date of filing: **10.07.87**

(51) Int. Cl.⁴: **C12N 9/64**, C12N 15/00, A61K 37/54

(30) Priority: **11.07.86 US 884357**

(43) Date of publication of application:
**20.01.88 Bulletin 88/03**

(84) Designated Contracting States:
**AT BE CH DE ES FR GB GR IT LI LU NL SE**

(71) Applicant: **NOVO INDUSTRI A/S**
**Novo Allé**
**DK-2880 Bagsvaerd(DK)**

(72) Inventor: **Wallén, Per Bruno**
**Trastvägen 9 G**
**S-902 37 Umea(SE)**
Inventor: **Norrman, Bo Anders**
**Skolgatan 87**
**S-902 46 Umea(SE)**
Inventor: **Nexo, Bjorn Andersen**
**Soborg Parkallé**
**10, DK-2860 Soborg(DK)**

(74) Representative: **Brown, John David et al**
**FORRESTER & BOEHMERT**
**Widenmayerstrasse 4/I**
**D-8000 München 22(DE)**

## (54) Modified tissue plasminogenen activator.

(57) Novel modified tissue plasminogen activators (t-PA) are described. The novel t-PA's are single-chained molecules being cleaved at one or more positions in the peptide sequence extending from Cys(409) to Cys(441) and capable of being activated in the presence of plasmin. The novel products are formed by cleavage of tissues plasminogen activators and are useful in treatment of vascular disorders.

EP 0 253 582 A1

Fig. 1

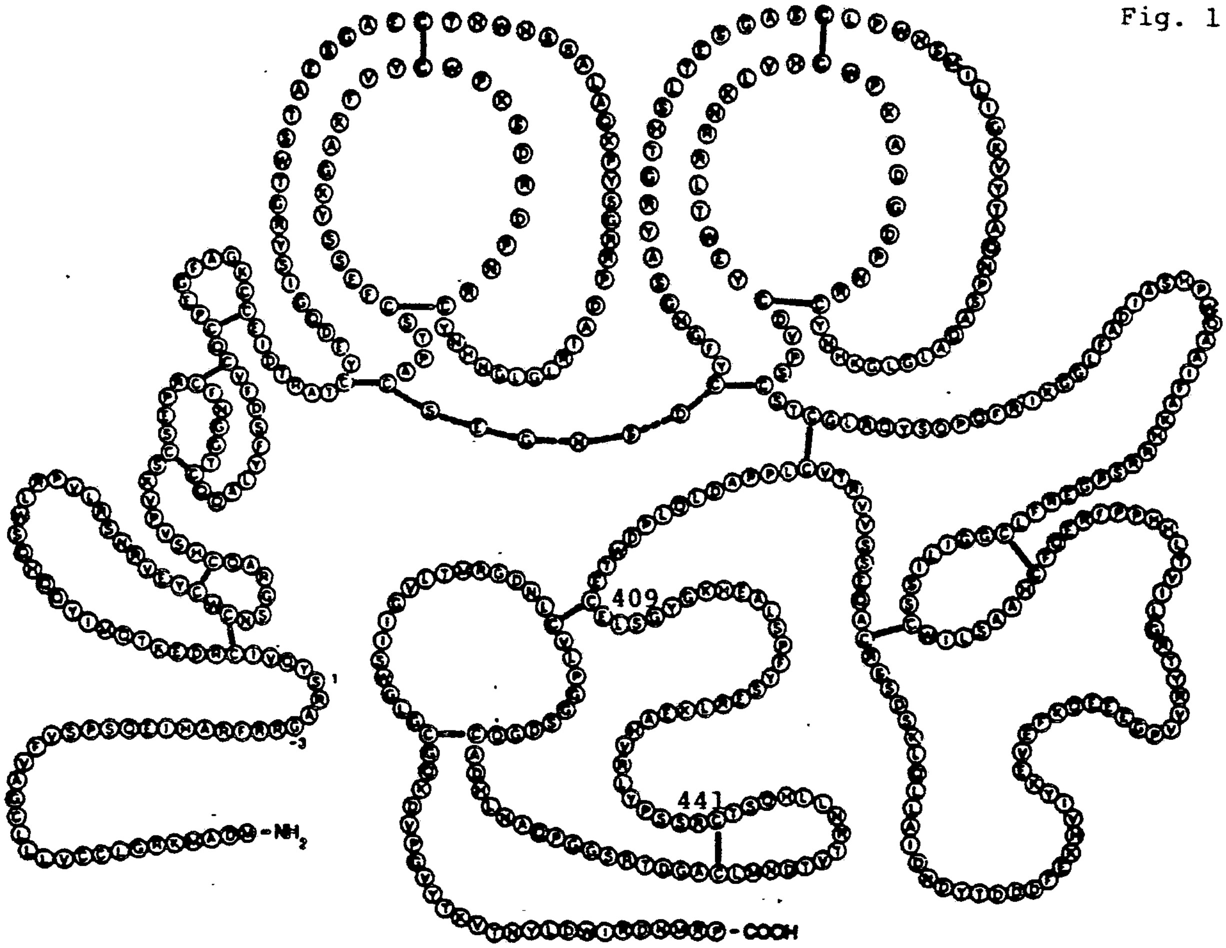

# MODIFIED TISSUE PLASMIN ACTIVATOR

The present invention relates to novel modified tissue plasminogen activators, methods for their preparation and pharmaceutical preparations containing such modified t-PA's.

## BACKGROUND OF THE INVENTION

Delicate enzymatic mechanisms control the fluidity of blood. Clotting factors ensure rapid coagulation of blood in response to injury by conversion of plasma fibrinogen to insoluble fibrin. Fibrinolytic enzymes are responsible for the orderly removal of already formed fibrin clots. If these mechanisms become deranged clots may form inappropriately inside the vascular bed. This can impair the local flow of blood and result in tissue malfunction, followed later by necrosis and replacement with scar tissue. Depending on the site of damage the injured individual will suffer from one of a variety of conditions, including myocardial infarction, deep vein thrombosis, pulmonary embolism, and stroke, all of which are serious public health problems.

Tissue damage does not occur instantaneously upon obstruction of the blood flow, rather it develops after some hours. Therefore, rapid restoration of the blood supply to the endangered area will minimize permanent damage. It is commonly accepted, that therapies ensuring the reopening of occluded vessels within a few hours have the potential to reduce the number of fatalities and permanent disabled persons appreciably, and much effort has been invested in the search for such techniques.

One approach to the problem has been to stimulate formation of the natural fibrinolytic enzymes. The major fibrinolytic enzyme of the body, plasmin, circulates in blood as the proenzyme plasminogen, from which it is formed by proteolytic cleavage of a single peptide bond between Arg(560) and Val(56l) by the action of plasminogen activators, e.g. tissue plasminogen activator t-PA (l-2). Plasminogen binds with high affinity to fibrin clots, and degrades fibrin to soluble fragments when converted to plasmin. Plasminogen activation is therefore a logical concept in fibrinolytic therapy.

However, plasmin is a nonspecific protease, and if it is formed in the circulating blood it will degrade a variety of plasma proteins, including fibrinogen, and the coagulations factors V and VIII. These reactions are initially held partly in check by $\alpha$-2 antiplasmin, a highly efficient stoichiometric inhibitor of plasmin present in plasma. As the inhibitor is depleted the systemic effects of plasmin become pronounced. Degradation of fibrinogen and clotting factors and accumulation of inactive fibrinogen split products make the blood coagulate inefficiently, a situation which can lead to serious episodes of bleeding. Generalized activation of plasmin is therefore hazardous. This is the central issue of fibrinolytic therapy: how is it possible to obtain efficient plasminogen activation and fibrin removal at the site of occlusion, without at the same time putting the patient in a generalized fibrinolytic state?

Several plasminogen activators have been tested in the clinic as fibrinolytic agents. Streptokinase is a protein derived from beta hemolytic streptococci, which binds to plasminogen. The complex formed is an active enzyme which converts plasminogen into plasmin. In other words, streptokinase turns plasminogen into an autoactivating enzyme. As streptokinase action is independent of the presence of a fibrin clot, the systemic side effects of streptokinase are pronounced. Moreover, because streptokinase is a bacterial protein it induces a prominent immune response. Some patients possess antibodies to it, either as a consequence of previous therapy or because of past streptococcal infections. This complicates determination of an effective therapeutic dose.

Urokinase is a serine protease occurring naturally in human urine. It is composed of 2 peptide chains connected with a disulfide bond. Urokinase is an efficient activator of plasmino gen. It also contains a structure, known as a kringle, which generally endows molecules with an affinity for fibrin. However, urokinase activity is not dependent on the binding to fibrin, and urokinase has not demonstrated any increased specificity for clot resolution in vivo or in vitro, but reproduces many of the systemic side effects known from streptokinase.

Prourokinase is a zymogen form of urokinase, in which the two chains of urokinase are still joined by a peptide bond. This molecule is essentially devoid of enzymatic activity. This molecule may have increased clot specificity, relative to urokinase itself. Presumably, this arises because conversion to active urokinase primarily occurs on the clot. The mechanism is unclear, since pro-urokinase per se seems to have low affinity for fibrin. The clinical value of the molecule remains to be seen.

Tissue plasminogen activator (t-PA) is also a serine protease, which under natural circumstances activates plasminogen. This activator differs from streptokinase and urokinase by its much stronger affinity to fibrin. Furthermore, the presence of fibrin will cause and up to I000-fold increase of the activation rate indicating the formation of a ternary activation complex between t-PA, plasminogen and fibrin (3, 4 and references therein). The t-PA is a 65,000 dalton glycoprotein, which occurs in two major forms, as a one chain molecule, and as a two-chain molecule in which the two chains are held together by a disulfide bond. These chains are designated A-chain for the polypeptide originating from the N-terminal part of the enzyme, and B-chain for the polypeptide from the C-terminal part of the enzyme (3). A schematic two-dimensional model of the human precursor t-PA protein including signal peptide and pro-sequence is shown in fig. I (20). This model assumes that the A-chain (the heavy chain) contains a finger domain, a growth factor-like domain and two kringle structures (kl and k2). The B-chain (the light chain) contains the active site for serine protease activity.

Conversion of one-chain to two-chain t-PA is catalyzed by plasmin and consists of cleavage of a single peptide bond between the amino acids Arg(275) and Ile(276) (5-8). This cleavage occurs efficiently on the surface of fibrin where plasmin normally is present. (Here and in the following, the numbering system of Pennica et al. has been followed (8)). Both forms of the molecule are enzymatically active, but the two-chain form has a somewhat higher specific activity. The factor of activation upon conversion to two-chain form varies with the assay system. Other variations of structure also exist in t-PA. For instance, some t-PA molecules start at Ser(l), while other molecules start at Gly(-3), and an approximately 3000 dalton molecular weight variation reflects the presence or absence of a glycosylation on Asn(l84).

Due to the strong ability of fibrin to bind t-PA and to stimulate the activation of plasminogen by this activator it has been anticipated that use of t-PA in thrombolytic treatment would eliminate the problems arising from systemic activation of plasmin. Preliminary experiments in animals as well as early treatments of patients with thrombosis and myocardial infarction seemed to support this idea (9). However, elaborate clinical trials have shown that the very high doses needed to give significant thrombolysis often give rise to side effects. Even if they are less pronounced than those given by streptokinase, substantial amounts of free plasminogen is converted to plasmin. This gives a reduction of plasma fibrinogen and the patients suffer from bleeding episodes.

The problem arises from the inherent enzymatic activity of t-PA, which allows circulating t-PA to act systemically prior to fibrin binding. It is aggravated by several other properties of t-PA. First of all, the halflife of t-PA in plasma in vivo is so short, that only a miniscule proportion of the injected molecules ever reach the fibrin clot. Secondly, inhibitors of t-PA are present in plasma and tissues and further reduce the chance for active t-PA to reach the intended target.

Presently three PA-inhibitors are known, one being isolated from endothelial cells (PAI-l), one from placenta (PAI-2), and the third one from urine (PAI-3). PAI-l is very potent in inhibiting both t-PA and urokinase, PAI-2 inhibits two-chain t-PA more efficiently than the one-chain t-PA. High plasma levels of t-PA inhibitors have been found in patients developing acute myocardial infarction (2l) as well as in patients with thrombotic disease (22). Since both these patient groups are target groups for t-PA treatment it would be desirable to have a modified human t-PA exhibiting a reduced affinity towards one or more of those inhibitors.

## SUMMARY OF THE INVENTION

The purpose of the present invention is to provide a plasminogen activator characterized by substantially reduced systemic activity, predominantly local effect on fibrin deposits, reduced affinity towards natural t-PA inhibitors and increased survival time in circulating blood.

The present invention is based on the surprising discovery that modified human t-PA with the above mentioned properties is obtained when one-chain human t-PA is proteolytically cleaved in a certain area in the t-PA molecule.

Such modified t-PA may be prepared by proteolytical treatment of one-chain human t-PA, preferably by means of proteases, such as chymotrypsin, Staph.aureus V8 protease, pepsin and Armellaria mellea protease. The t-PA may also be modified by suitable chemical treatment.

One-chain t-PA for use in the present invention may be derived from any suitable source, preferably human, e.g. human tissue, blood, serum and cell cultures. For instance t-PA may be derived from human uterine tissue or from a human melanoma cell line (see EP patent application No. 004l766). The t-PA source may also be prepared by recombinant DNA-technology, e.g. as described in EP patent application No. 00936l9.

The present invention furthermore embraces use of modified one-chain t-PA, e.g. as described in PCT patent application No. US85/01613, (WO No. 86/01538) and EP patent applications No. 196,920 and 207,589. In WO patent application No. 86/01538 single-chained t-PA is described in which Lys(277) is replaced by another amino acid, e.g. Ile. EP patent applications No. 196,920 describes modified t-PA's comprising intact B-chain connected to kringle 2, and EP patent application No. 207,589 describes t-PA modified in the growth factor domain or lacking this domain. t-PA as used herein shall embrace all of the foregoing forms.

The area in the one-chain t-PA molecule susceptible to proteolytic cleavage according to the present invention extends from Cys(409) to Cys(441) in the B-chain of human t-PA.

According to the present invention any cleaving agent (e.g. protease) which cleaves in the Cys(409)-Cys(441) region may be used. Proper cleavage can be determined either by sequence analysis or by testing to see if the cleaved t-PA functions in the method of the invention.

According to the present invention it has been shown that treatment of one-chain t-PA with a cleaving agent, preferably chymotrypsin, causes a marked decrease in both the amidolytic activity and plasminogen activation. By treatment of the chymotrypsin cleaved one-chain t-PA with plasmin to generate cleavage at Arg(275), amidolytic activity of the twice cleaved molecule was largely restored. Moreover two-chain t-PA (i.e. plasmin cleaved one-chain t-PA) retains both its amidolytic activity and plasminogen activating activity after chymotrypsin cleavage in the same area in the B-chain thus evidencing that the twice cleaved one-chain t-PA has regained its plasminogen activating activity as well. Thus chymotrypsin treatment of one-chain t-PA generates an inactive t-PA i.e. a modified t-PA with substantially reduced systemic activity, which is capable of being activated with plasmin, i.e. on the surface of fibrin and thus exhibits a predominantly local effect on fibrin deposits.

t-PA activity is often measured as amidolytic activity and plasminogen activating activity, respectively. Amidolytic activity, i.e. the general catalytic activity, may be measured by means of an assay comprising a chromogenic low molecular weight substrate D-Ile-Pro-Arg-pNA (S-2288) which is cleaved directly by t-PA and offers a convenient colorimetric determination of the amidolytic activity.

Plasminogen activating activity can be determined indirectly in an assay which contains plasminogen and a chromogenic substrate D-Val-Phe-Lys-pNA which is cleaved by plasmin. In this assay colour development follows an accelerating parabolic curve as plasmin accumulates. The acceleration constant is proportional to the t-PA activity. As this assay determines the proteolytic activity of t-PA on the natural substrate plasminogen it is more closely related to the fibrinolytic effect. In both types of assay it is possible to include fibrin, so as to study its stimulation of t-PA enzyme activity. For present purposes activity or inactivity of the t-PA shall be measured in accordance with the foregoing assays.

## BRIEF DESCRIPTION OF THE DRAWINGS

The present invention is described below in greater detail with reference to the accompanying drawings in which

fig. 1 shows a schematic two dimensional model of the human precursor t-PA protein including signal peptide and pro-sequence,

fig. 2 shows the amidolytic activity and plasminogen activating activity of chymotrypsin digested one-chain t-PA,

fig. 3 shows the amidolytic activity of chymotrypsin digested one-chain t-PA before and after plasmin treatment, and

fig. 4 shows amidolytic activity and plasminogen activating activity of chymotrypsin treated two-chain t-PA.

## DETAILED DESCRIPTION OF THE INVENTION

In its broadest aspect the present invention provides novel modified t-PA for use as a fibrinolytic agent characterized in being cleaved at one or more positions in the peptide sequence extending from Cys(409) to Cys(441) in the t-PA molecule and capable of being activated in the presence of plasmin.

The present invention embraces all modified t-PA molecules cleaved in the (409)-(441) region which are substantially less active than native t-PA but which in the presence of plasmin, in particular plasmin associated with fibrin, regain or increase their activity.

The modified t-PA according to the present invention is preferably of human origin.

The modified t-PA may be further modified in the A-chain, i.e. in the fibrin binding domain as described above provided such further modifications have no substantial effect on characteristics of the modified t-PA according to the present invention. Such further modifications includes removals and or modifications of the finger and/or growth factor sequences and/or kringles I or 2.

According to a preferred embodiment of the present invention the modified t-PA has been cleaved at peptide bonds 420 - 42l and/or 423 - 424.

The modified t-PA according to the present invention may be characterized as containing a peptide sequence comprising n amino acid residues linked to Cys(409) and a peptide sequence comprising m amino acid residues linked to Cys(44l), n and m being integers from 0 to about 50. Preferably n + m ≦ 50 and more preferably n + m ≦ 3l.

More specifically the modified t-PA may contain a peptide sequence linked to Cys(409) consisting of n amino acid residues from the N-terminal end of the peptide sequence II

-Glu-Leu-Ser-Gly-Tyr-Gly-Lys-His-Glu-Ala-Leu-Ser-Pro-Phe-Tyr-Ser-Glu-Arg-Leu-Lys-Glu-Ala-His-Val-Arg-Leu-Tyr-Pro-Ser-Ser-Arg-, (II)

and a peptide sequence linked to Cys(44l) consisting of m amino acid residues from the C-terminal end of the peptide sequence II, n and m being integers from 0 to 3l and n + m ≦ 3l.

Referring to the two-dimensional structure as shown in fig. I the modified t-PA according to the present invention can be expressed by the general formula I:

```
Q-Cys-X_n          Y_m-R-Cys-Z
   |                      |
   |_______ S-S __________|                (I)
```

wherein $X_n$ is a peptide sequence comprising n amino acid residues,
$Y_m$ is a peptide sequence comprising m amino acid residues, n and m are integers from 0 to 50, Q is the human t-PA sequence from Gly(-3) or Ser(l) to Glu(408) or a modified form of such sequence, including proteolytic degradation products thereof, R is the human t-PA sequence from Cys(44l) to Val(483) and Z is the human t-PA sequence from Leu(485) to Pro(527) or proteolytic degradation products thereof (assuming such degradation does not substantially affect the enzymatic characteristics of the modified t-PA in the presence of plasmin).

Preferably in formula (I) n + m ≦ 50 and more preferably n + m ≦ 3l.
Preferably $X_n$ is a peptide sequence comprising n amino acid residues from the N-terminal end of the peptide sequence II

-Glu-Leu-Ser-Gly-Tyr-Gly-Lys-His-Glu-Ala-Leu-Ser-Pro-Phe-Tyr-Ser-Glu-Arg-Leu-Lys-Glu-Ala-His-Val-Arg-Leu-Tyr-Pro-Ser-Ser-Arg-, (II)
and $Y_m$ a peptide sequence comprising m amino acid residues from the C-terminal end of the peptide sequence II, n and m being integers from 0 - 3l and n + m ≦ 3l.

In the above formula (I) n and m may preferably be ll and 20, l4 and l7 or ll and l7, respectively. Furthermore, when n is l, then m may be 30, 22, l4 or l0. Also, when n is 9, then m may be 22, l4 or l0, when n is l7, then m may be l4 or l0 and when n is 2l then m may be l0.

Sequence II might have a single or small number of amino acid substitutions without substantial loss of utility of the molecule and the present invention embraces such substituted forms.

In the above formula I the sequence Q may be modified as described above. These modifications include removals and/or modifications of the finger and/or growth factor sequences and/or kringles I or 2.

The simultaneous decrease in amidolytic and fibrinolytic activity by the chymotrypsin treatment indicates that the chymotryptic cleavage affects the active site or active site-related regions of one-chain t-PA. SDS-poly-acrylamide gel electrophoresis under reducing conditions of chymotrypsin treated one-chain t-PA revealed that two pairs of new bands had appeared, with an $M_r$ or about 45/50,000 and of about l7/20,000 respectively. N-terminal sequence analysis identified cleavage sites at peptide bonds 420-42l and 423-424 in the t-PA B-chain. These cleavages seem to disrupt the conformation of the B-chain in such a way that its enzymatic activity is lost.

As treatment of samples of chymotrypsin-digested one-chain activator with plasmin largely restores amidolytic activity, chymotrypsin by cleaving bonds 420-421 and 423-424 seems to convert the active one-chain activator t-PA into an "inactive" zymogen (modified t-PA) which may then be reactivated by plasmin cleavage at Arg(275).

Confirmatory data obtained by a third party subsequent to the chymotrypsin work which is the basis in part for this patent filing substantiated that t-PA, cleaved in the 409-441 region (at 418-419 or 426-427) by Staph. aureus V8 protease, is activated by treatment with plasmin.

The invention embraces the use of any cleaving agent (preferably a protease) which cleaves in this area to give an inactive t-PA which becomes active in the presence of plasmin, in particular plasmin associated with fibrin, i.e. at the locus of the fibrin clot.

Minor further proteolytic degradations of the remaining t-PA molecule might occur. So far as this has no deleterious effect on the goal of the present invention such slightly further modified t-PA is contemplated to be covered by the invention.

According to its second aspect the present invention provides a method for the production of modified t-PA by which method the corresponding one-chain t-PA is subjected to a proteolytic cleavage in the peptide sequence extending from Cys(409) to Cys(441) in the B-chain.

According to a preferred embodiment of the present invention the proteolytic cleavage is performed by means of a suitable protease, preferably chymotrypsin, Staph. aureus V8 protease or pepsin. The chymotrypsin cleavage may preferably be conducted by means of an immobilized enzyme at pH 7.5-8.5 and 20-30°C. Any source of the chymotrypsin may be used. Sepharose-coupled chymotrypsin is preferably meticulously freed from traces of trypsin activity by treatment with N-p-tosyl-L-lysine chloromethylketone. The cleavage may be performed by incubating 1 mg t-PA (dissolved in 20 ml 0.18 M $NH_4HCO_3$ pH 8.1 containing 0.01 % Tween 80) with 20 mg Sepharose derivative (40 µg chymotrypsin) for about 8 h at 25°C.

The cleavage with pepsin and Staph. aureus V8 protease may be conducted by use of the free enzymes at acid pH, preferably pH 2 and pH 4, respectively.

According to a third aspect of the present invention there is provided a fibrinolytic pharmaceutical preparation comprising an effective amount of a modified t-PA according to the present invention.

Such preparations are preferably buffered solutions containing suitable pharmaceutically acceptable carriers and adjuvants. The aqueous solution is sterilized, e.g. by filtering the solution through a membrane filter having a pore size sufficiently small to retain cellular microbes. The pharmaceutical preparations may be used and administered in the same manner and similar dosage ranges as conventional t-PA.

According to a fourth aspect of the present invention there is provided a method for treating patients having vascular disorders comprising administering to the patient an effective amount of a modified t-PA as described above.

## Examples

Native one-chain human tissue plasminogen activator (t-PA) was purified by immunoaffinity chromatography and gel filtration (6). A Sepharose-bound monoclonal antibody with low affinity for two-chain t-PA was used in the immunosorbent step (1:3 C:5, available as PAM I from BioPool AB, Umeå, Sweden). The specific activity was determined by clot-lysis assay (5), and was found to be 850,000 units/mg, when compared to the WHO international t-PA-standard. Two-chain activator was prepared by digestion of one-chain activator with plasmin-Sepharose (6).

Plasminogen was prepared from fresh frozen plasma by affinity chromatography (10-12). Plasmin was generated by Sepharose-urokinase treatment (5). Fibrinogen was prepared from fresh frozen citrated human plasma (13) and passed through a Sepharose-lysine column to remove traces of plasminogen (14). Fibrin I monomers (desAA fibrin monomers) were prepared as described (12).

Chromogenic peptide substrates for plasmin (H-D-Val-Phe-Lys-pNA, S-2390), chymotrypsin (MeO-Suc-Arg-Pro-Tyr-pNA, S-2586), trypsin (Benzoyl-Ile-Glu-Gly-Arg-pNA, S-2222) and t-PA (H-D-Ile-Pro-Arg-pNA, S-2288) were purchased from Kabi Vitrum, Stockholm, Sweden. The substrates were dissolved in distilled water to a concentration of 5 mM.

TosLys$CH_2$Cl-treated chymotrypsin, purified monoclonal antibody, and plasmin were coupled to Sepharose-4B (Pharmacia, Sweden) by the CNBr-method (16). Incorporation of proteins was found to be 2 mg/g gel for Sepharose-IgG, 1.5 mg/g for Sepharose-plasmin and 2 mg/g gel for Sepharose-chymotrypsin.

The specific activity of chymotrypsin was determined with chymotrypsin specific substrates (S-2586). The substrate concentration was 2*Km. The activity was equivalent to 80 U/g gel.

Additional treatment with Nα-p-tosyl-L-lysine chloromethylketone was used to remove traces of trypsin activity still present in the Sepharose-chymotrypsin. I g of Sepharose-chymotrypsin was dispersed in I5 ml 0.I5 M ammonium bicarbonate and I.2 ml $TosLysCH_2Cl$ solution (3 mg/ml in methanol) was added. The mixture was incubated for 8 h at +4°C and subsequently washed in 0.I5 M ammonium bicarbonate, distilled water, and 0.04% sodium azide. The gel was then suction-dried and stored at +4°C.

In the present specification the following abbreviations are used:

t-PA: tissue plasminogen activator
DFP: diisopropylfluorophosphate
SDS: Sodium dodecyl sulphate
$TosLysCH_2Cl$: N-p-tosyl-L-lysine chloromethylketone
$TosPheCH_2Cl$: N-p-tosyl-Lphenylalanine chloromethylketone
Buffer A: 0.06 M Tris buffer, adjusted to pH 6.8 with HCl and containing I2% glycerol, I.2% SDS and 0.I% bromophenol blue dye
NaCl/Pi: Phosphate buffered saline; 50 mM phosphate, I00 mM NaCl (I=0.I5), pH 7.3 with 0.I g/l Triton X-I00

Example I

Chymotrypsin treatment of one-chain t-PA and subsequent plasmin treatment of the chymotrypsin-cleaved product

For digestion of t-PA with chymotrypsin stock solutions of t-PA were diluted in I M ammonium bicarbonate to a concentration of 290 µg/ml and further diluted I:5.5 in distilled water containing 0.0I% Tween 80, yielding a final concentration of about 50 µg/ml. An aliquot was drawn, representing the starting material, and Sepharose-chymotrypsin (2 mg) was added per I00 µg protein. The reaction mixture was incubated during continuous end-over-end rotation at room temperature. After I5 min., 30 min., I h, 2 h, 4 h, 8 h and 24 h, the incubation vessel was centrifuged, and an aliquot was drawn and filtrated through a 0.45 um filter (HV 4, Millipore). Two 25 µl aliquots of each sample were lyophilized for SDS-polyacrylamide gel electrophoresis, and the remaining material was stored at -90°C.

Amidolytic activity of the chymotrypsin treated t-PA was determined with the substrate H-D-Ile-Pro-Arg-pNA (S-2288). Samples from digestion experiments were diluted I:20 and I:40, respectively in NaCl/Pi and I00 µl of diluted samples was mixed with I00 µl 0.8 mM S-2288 in the wells of a microtiter plate. For analysis in the presence of fibrin, 2 µl fibrin I monomer (final concentration 0.3 µM) was added to the wells (I7). A Titertek Multiskan from Flow Laboratories was used for spectrophotometric determinations. The absorbance at 405 nm and, in order to correct for the turbidity caused by the fibrin, at 492 nm were measured every I0 minutes. The turbidity corrected absorbance $A_{405}$-$A_{492}$ was calculated and from this the amidolytic activity/min was determined.

Plasminogen activating activity was measured with a chromogenic substrate assay (I8). In the wells of a microtiter plate, I00 µl sample (diluted I:40 and I:320,000, respectively in NaCl/Pi) was mixed with I00 µl reagent, containing 2 µM Glu-plasminogen and I mM H-D-Val-Phe-Lys-pNA (S-2390). For determinations in the presence of fibrin, 2 µl of fibrin I monomer was added. Turbidity corrected absorbance was determined as described above. To ensure determinations in the linear range of the assay, absorbancies between 0.I5 and 0.4 were chosen. An average rate of plasminogen activating was calculated by dividing the absorbancy value with that of the time squared ($A/h^2$) (I8).

Amino acid sequence analysis was performed on peptide fragments eluted from polyacrylamide gels, using an electroelution concentrator (C.B.S. Scientific Company, Inc. Del Mar, USA). The elution buffer was 0.05 M ammonium bicarbonate containing 0.I% SDS. Samples (0.5-2 ml) containing 0.2-I nmole peptide were filtered, concentrated under nitrogen and loaded as 50 µl aliquots into an Applied Biosystems 470A gas phase sequencer, programmed according to the manufactorers' instructions. Phenylthiohydantoin derivatives were analyzed with high-performance liquid chromatography (7,I9).

Chymotrypsin treatment caused a continuous decrease in the amidolytic activity as shown in fig. 2 in which residual activity, expressed as percent of that in the starting material is plotted against time of chymotrypsin digestion. After 2 h digestion, the activity had decreased to 50% and after 8 h, l0% of the amidolytic activity remained. Plasminogen activating activity, measured both in the presence and absence of fibrin, decreased in a similar manner. No marked changes in either amidolytic activity or plasminogen activation were found, when one-chain t-PA was incubated for 8 h during the same conditions without chymotrypsin.

SDS-polyacrylamide gel electrophoresis of chymotrypsin-treated t-PA was performed after reduction. A double band corresponding to $M_r$ 45/50.000 could be detected after l5 min., together with two new bands with an $M_r$ of about l7,000 and 20,000 respectively. After further digestion, the band corresponding to intact one-chain activator disappeared.

The fragments corresponding to the major bands were eluted from the polyacrylamide gel and subjected to N-terminal amino acid sequence analysis. At least l0 residues were identified for each fragment. The 45/50,000 fragments contained the N-terminal part of the native protein. The l7/20,000 fragments were found to originate from the B-chain and the new N-terminal sequence of these fragments started at Tyr(424) of the native protein. A minor portion, about l5% of the l7,000 fragment started at Ser-(42l).

Plasmin treatment of chymotrypsin digested one-chain t-PA was performed with 30 µl aliquots, diluted l:l0 in 0.l5 M ammonium bicarbonate containing 0.0l% Tween 80. Sepharose-plasmin (6 mg) was added and after end-over-end rotation for 45 min. at room temperature, l00 µl of the incubation solution was filtered through a 0.45 m filter. After dilution in NaCl/Pi, the amidolytic activity was immediately assayed.

Treatment of chymotrypsin digested one-chain t-PA with plasmin causes a marked increase in the amidolytic activity as shown in fig. 3 in which amidolytic activity of samples (dilution l:20) before plasmin treatment (dark area) and after plasmin treatment (light area) is expressed as per cent of amidolytic activity of non-treated starting material. Numbers above bars show the relative increase for each sample. Even after 8 h chymotryptic digestion, the amidolytic activity after plasmin treatment increased to a level corresponding to that of the starting material (before chymotrypsin treatment).

Accordingly, it was demonstrated that plasmin treatment is capable of reactivating the "inactive" modified t-PA.

Example 2

Chymotrypsin treatment of two-chain t-PA

Two-chain t-PA was digested with chymotrypsin under the same conditions as for the one-chain t-PA in example l. Amidolytic activity and plasminogen activation of chymotrypsin treated two-chain t-PA were determined in the presence and absence of fibrin. For measurements of amidolytic activity, samples were diluted l:l20. For measurements of plasminogen activation, samples were diluted l:200 and l:320,000, respectively. The results are shown in fig. 4 in which residual activity, expressed as percent of that in the starting material is plotted against time of chymotrypsin digestion. A marked difference from one-chain t-PA was found. Amidolytic activity remained high, even after 8 h. Plasminogen activation activity remained considerably higher than when one-chain t-PA was treated with chymotrypsin.

Electrophoretic analysis revealed that l7/20,000 fragments described earlier were formed also by treatment two-chain t-PA with chymotrypsin. Thus chymotrypsin treatment of two-chain t-PA generates the same molecule as plasmin treatment of chymotrypsin modified one-chain t-PA.

Accordingly, evidence is provided that plasmin treatment of chymotrypsin modified one-chain t-PA also can function to restore plasminogen activating activity.

The features disclosed in the foregoing description, in the following claims and/or in the accompanying drawings may, both separately and in any combination thereof, be material for realising the invention in diverse forms thereof.

REFERENCES

l. Collen, D. (l980) Throm.Haemostas. 43, 77-89.

2. Wallén, P. (l980) In Fibronolysis (Kline DL and Reddy KNN, Eds.) CRC-Press, Boca Raton, Florida, pp. l-24.

3. Wallén, P. (I977) in Thrombosis and urokinase (Paoletti, R. & Sherry, S., eds.) 9, pp. 9I-I02, Academic Press, London.

4. Rånby, M. and Wallén, P. (I985) in Thrombolysis. Biological and therapeutic properties of new thrombolutic agents (Eds. Collen, D., Lijnen, H.R. & and Verstraete, M.) Churchill Livingstone, Edingburgh pp. 3I-48.

5. Wallén, P., Bergsdorf, N. & Rånby, M. (I982) Biochim.Biophys. Acta 7I9, 3I8-328.

6. Wallén, P., Pohl, G., Bergsdorf, N., Rånby, M., Ny, T. & Jörnvall, H. (I983) Eur.J.Biochem. I32, 68I-686.

7. Pohl, G., Källström, M., Bergsdorf, N., Wallén, P. & Jörnwall, H. (I984) Biochemistry 23, 370I-3707.

8. Pennica, D., Holmes, W.E., Kohr, W. J., Harkins, R.N., Vehar, G.A., Ward, C.A., Bennet, W.F. & Collen, D. (I983) Nature 30I, 2I4-22I.

9. Van de Werf et al. (I984), New England, Journal of Medicine 3I0 (I984), 609-6I3.

I0. Deutsch, D.G. & Mertz, E.T. (I970) Science I70, I095-I096.

II. Wiman, B. & Wallén, P. (I973) Eur.J.Biochem. 36 25-3I.

I2. Wallén, P. & Wiman, B. (I972) Biochim.Biophys.Acta 257, I22-I34.

I3. Blombäck, B. & Blombäck, M. (I956) Ark.Kemi. I0, 4I5-443.

I4. Matsuda, M., Iwanaga, S. & Nakamura, S. (I972) Thromb. Res. I, 6I9-630.

I5. Norrman, B., Wallén, P. & Rånby, M. (I985) Eur.J.Biochem. I49, I93-200.

I6. Axén, R., Porath, J. & Ernback, S. (I967) Nature (Lond.) 2I4, I302-I304.

I7. Rånby, M., Suensen, E., Bergsdorf, N., Norrman, B. & Wallén, P. (I983) Prog.Fibrinolys. 6, I82-I84.

I8. Rånby, M., Norrman, B. & Wallén, P. (I982) Thromb.Res. 27 743-749.

I9. Zimmerman, C.L., Appella, E. & Pisano, J.J. (I977) Anal.Biochem. 77 569-573.

20. (Ny et al., Proc. Natl.Acad.Sci. USA 8I(I984), 5358).

2I. Hamsten, A., Wiman, B., de Faire, U. and Blombäck, M.: Increased plasma levels of a rapid inhibitor of tissue plasminogen activator in young survivors of myocardial infarction. N. Engl. J. Med. 3I3 - (I985) I557-63.

22. Nilsson, I.M., Ljungnér, H. and Tengborn, L.: Two different mechanisms in defective fibrinolysis: low concentration of plasminogen activator or increased concentration of plasminogen activator inhibitor. Brit.Med.J. 290(I985) I453-56.

## Claims

I. Modified t-PA for use as a fibrinolytic agent characterized in being cleaved at one or more position in the sequence from Cys(409) to Cys(44I), said t-PA being essentially fibrinolytically inactive, but capable of being activated in the presence of plasmin.

2. Modified t-PA according claim I, said modified t-PA being of human origin.

3. Modified t-PA according to claim 2, being further modified in the fibrin binding domain.

4. Modified t-PA according to claim 3, being further modified in the finger domain.

5. Modified t-PA according to claim 3, lacking the finger and growth factor sequences.

6. Modified t-PA according to claim 3, further lacking kringle I.

7. Modified t-PA according to claim 3 being modified in or lacking the growth factor sequence.

8. Modified t-PA according to any one of the previous claims being cleaved at peptide bonds 420-42I and/or 423-424.

9. Modified t-PA according to any of the previous claims I - 7 containing a peptide sequence comprising n amino acid residues linked to Cys(409) and a peptide sequence comprising m amino acid residues linked to Cys(44I), n and m being integers from 0 to about 50.

I0. Modified t-PA according to claim 9, wherein $n + m \leq 50$.

II. Modified t-PA according to claim 9, wherein $n + m \leq 3I$.

I2. Modified t-PA according to claim 9 wherein the peptide sequence linked to Cys(409) consists of n amino acid residues from the N-terminal end of the peptide sequence II

-Glu-Leu-Ser-Gly-Tyr-Gly-Lys-His-Glu-Ala-Leu-Ser-Pro-Phe-Tyr-Ser-Glu-Arg-Leu-Lys-Glu-Ala-His-Val-Arg-Leu-Tyr-Pro-Ser-Ser-Arg-, (II)

and the peptide sequence linked to Cys(44I) consists of m amino acid residues from the C-terminal end of the peptide sequence II, n and m being integers from 0 to 3I and $n + m \leq 3I$.

I3. Modified t-PA according to any of the previous claims I - 7 having the general formula I

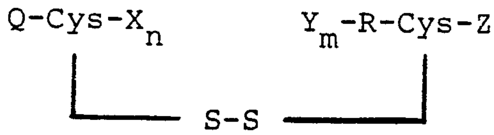

(I)

wherein $X_n$ is a peptide sequence comprising n amino acid residues, $Y_m$ is a peptide sequence comprising m amino acid residues, n and m being integers from 0 to about 50, Q is the human t-PA sequence from Gly(-3) or Ser(l) to Glu(408) or a modified form of such sequence including proteolytic degradation products thereof, R is the human t-PA sequence from Cys(44l) to Val(483) and Z is the human t-PA sequence from Leu(485) to Pro(527) or proteolytic degradation products thereof.

l4. Modified t-PA according to claim l3, wherein n + m ≤ 50.

l5. Modified t-PA according to claim l3, wherein n + m ≤ 3l.

l6. Modified t-PA according to claim l3, wherein $X_n$ is a peptide sequence comprising n amino acid residues from the N-terminal end of the peptide sequence II

-Glu-Leu-Ser-Gly-Tyr-Gly-Lys-His-Glu-Ala-Leu-Ser-Pro-Phe-Tyr-Ser-Glu-Arg-Leu-Lys-Glu-Ala-His-Val-Arg-Leu-Tyr-Pro-Ser-Ser-Arg-, (II)

$Y_m$ is a peptide sequence comprising m amino acid residues from the C-terminal end of the peptide sequence II, n and m being integers from 0 to 3l and n + m ≤ 3l.

l7. Modified t-PA according to claims l2 or l6, wherein n is ll and m is 20.

l8. Modified t-PA according to claims l2 or l6, wherein n is l4 and m is l7.

l9. Modified t-PA according to claims l2 or l6, wherein n is ll and m is l7.

20. Modified t-PA according to claims l2 or l6, wherein n is l and m is 30, 22, l4 or l0.

2l. Modified t-PA according to claims l2 or l6, wherein n is 9 and m is 22, l4 or l0.

22. Modified t-PA according to claims l2 or l6, wherein n is l7 and m is l4 or l0.

23. Modified t-PA according to claims l2 or l6, wherein n is 2l and m is l0.

24 The use of a modified t-PA according to any of the previous claims in the preparation of a fibrinolytic composition.

25. A process for the production of modified t-PA according to any of the previous claims l - 23 comprising subjecting the corresponding one-chain t-PA to a cleavage in the peptide sequence extending from Cys(409) to Cys(44l).

26. A process according to claim 25, wherein the one-chain t-PA is human one-chain t-PA.

27. A process according to claims 25 or 26, wherein the cleavage is conducted with a protease.

28. A process according to claim 27, wherein the protease is chymotrypsin.

Fig. 1

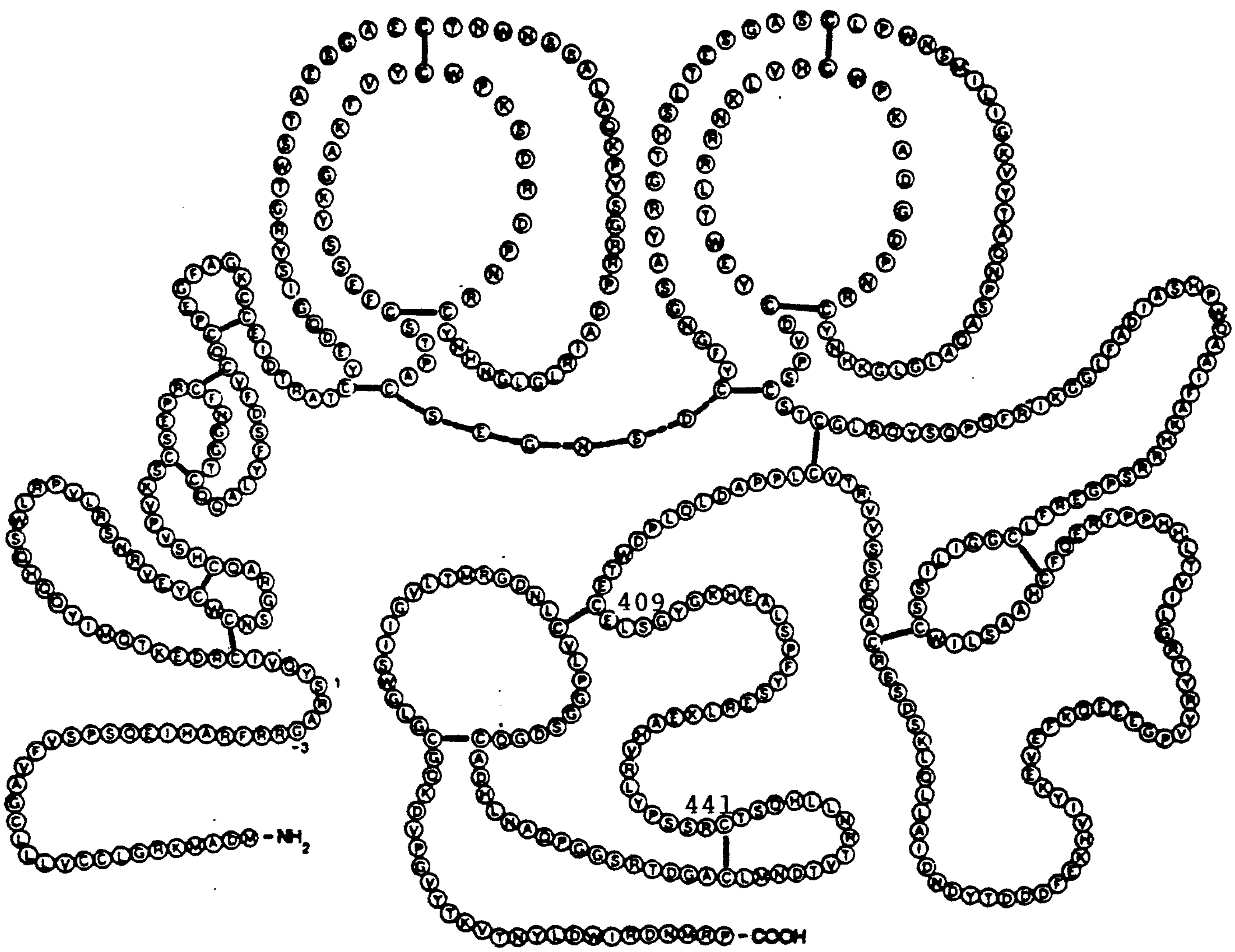

Fig. 2

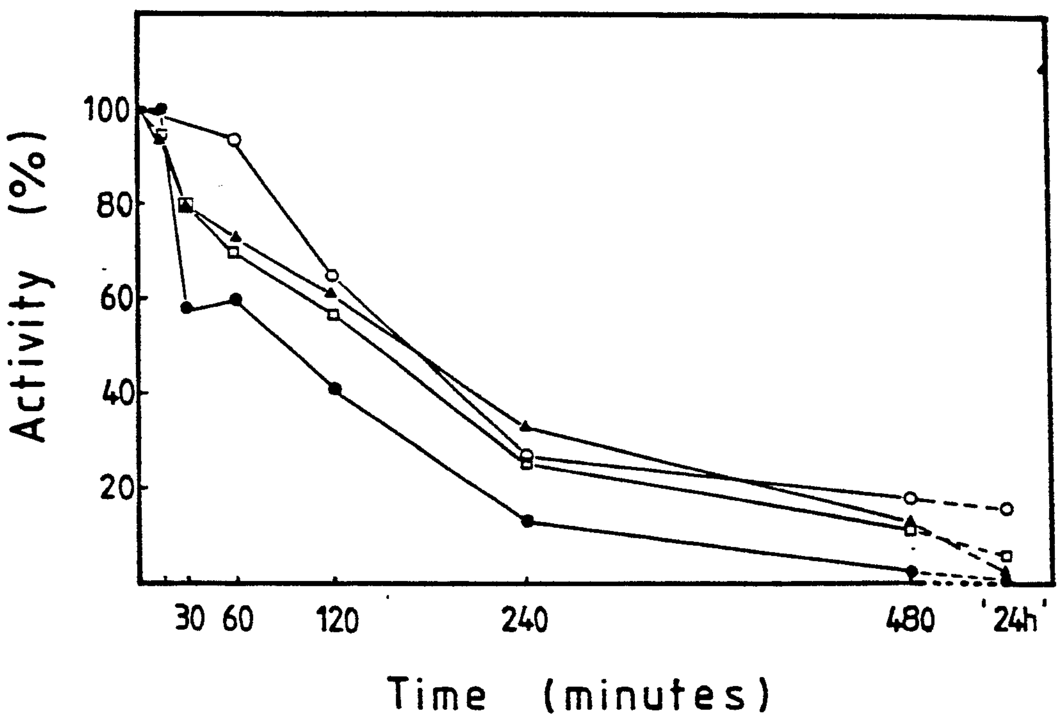


Amidolytic activity in the absence (□) and presence (▲) of fibrin. Plasminogen activation in the absence (○) and presence (●) of fibrin.

Fig. 3

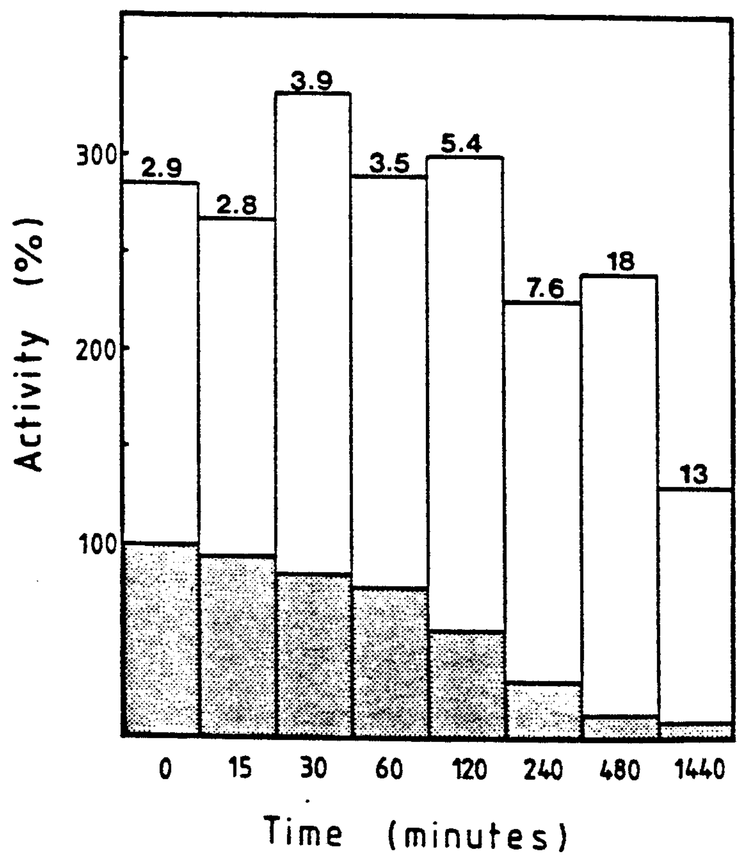


Amidolytic activity of samples (dilution 1:20) before (dark area) and after (light area) plasmin treatment is expressed as percent of non treated starting material. Numbers above bars show the relative increases for each sample.

Fig. 4

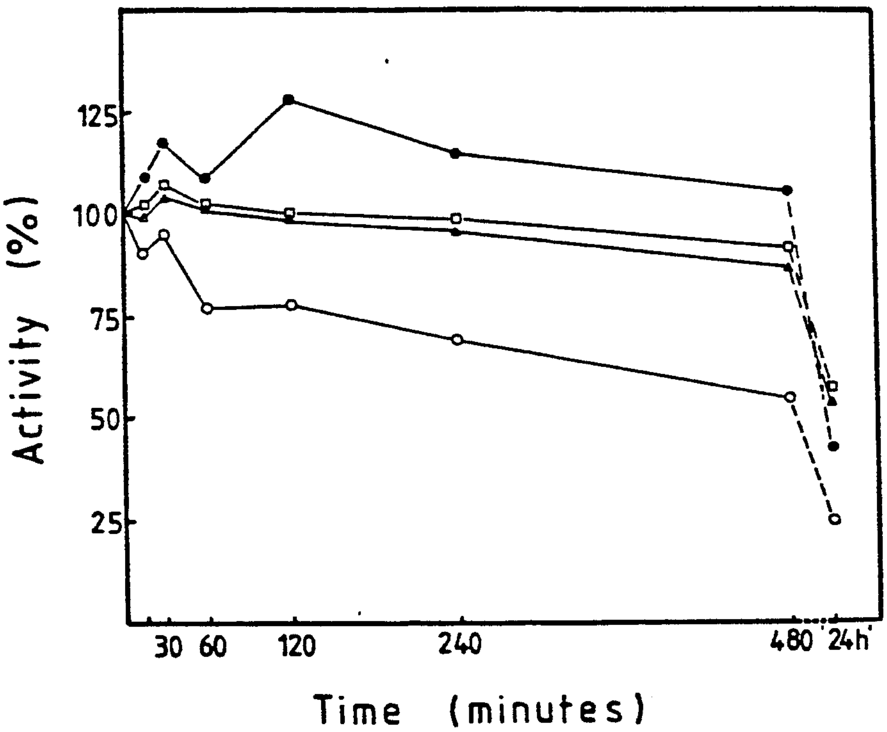


Amidolytic activity in the absence (□) and presence (▲) of fibrin. Plasminogen activation in the absence (○) and presence (●) of fibrin.

European Patent Office

# EUROPEAN SEARCH REPORT

Application number

EP 87 30 6099

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP-A-0 093 619 (GENENTECH, INC.) * Page 9, lines 19-33; page 10, line 19 - page 11, line 9; claims; figures 5,12 * --- | 1 | C 12 N 9/64<br>C 12 N 15/00<br>A 61 K 37/54 |
| D,A | WO-A-8 601 538 (BIOGEN N.V.) * Abstract; page 4, line 8 - page 5, line 9; page 7, line 14 - page 8, line 30 * --- | 1 | |
| A | THE JOURNAL OF BIOLOGICAL CHEMISTRY, vol. 257, no. 6, 25th March 1982, pages 2920-2925, US; D.C. RIJKEN et al.: "Fibrinolytic properties of one-chain and two-chain human extrinsic (tissue-type) plasminogen activator" * Abstract; discussion * --- | 1 | TECHNICAL FIELDS SEARCHED (Int. Cl.4)<br>C 12 N |
| P,X | CHEMICAL ABSTRACTS, vol. 105, no. 13, 29th September 1986, page 320, abstract no. 111040u, Columbus, Ohio, US; B. NORRMAN et al.: "Proteolytically induced variations in the enzymic properties of tissue plasminogen activator. Activations, inactivations and reactivations", & EUR. J. BIOCHEM. 1986, 159(1), 7-13 * Abstract * ----- | 1-28 | |

The present search report has been drawn up for all claims

| Place of search | Date of completion of the search | Examiner |
|---|---|---|
| THE HAGUE | 27-10-1987 | YEATS S.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03.82